(19) Europäisches Patentamt
European Patent Office
Office européen des brevets

(11) Veröffentlichungsnummer: **0 357 953 B1**

(12) **EUROPÄISCHE PATENTSCHRIFT**

(45) Veröffentlichungstag der Patentschrift: **07.04.93**

(51) Int. Cl.5: **C07C 211/63**, F15D 1/00

(21) Anmeldenummer: **89114244.0**

(22) Anmeldetag: **02.08.89**

# (54) Mischungen von organischen Ammonium-Salzen und deren Verwendung als Strömungsbeschleuniger.

(30) Priorität: **11.08.88 DE 3827183**

(43) Veröffentlichungstag der Anmeldung:
**14.03.90 Patentblatt 90/11**

(45) Bekanntmachung des Hinweises auf die Patenterteilung:
**07.04.93 Patentblatt 93/14**

(84) Benannte Vertragsstaaten:
**AT BE DE FR GB IT NL SE**

(56) Entgegenhaltungen:
**EP-A- 0 097 926**
**EP-A- 0 118 089**
**EP-A- 0 149 172**

(73) Patentinhaber: **HOECHST AKTIENGESELLSCHAFT**
**Postfach 80 03 20**
**W-6230 Frankfurt am Main 80(DE)**

(72) Erfinder: **Ohlendorf, Dieter, Dr.**
**Am Kühlen Grund 4**
**W-6237 Liederbach(DE)**
Erfinder: **Hofinger, Manfred, Dr.**
**Rossfeldstrasse 7a**
**W-8269 Burgkirchen(DE)**
Erfinder: **Wehle, Detlef, Dr.**
**Ahornstrasse 8**
**W-8261 Kastl/Obb(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Es ist bereits bekannt, daß quartäre Ammoniumsalze, die eine langkettige Alkylgruppe enthalten, als Strömungsbeschleuniger eingesetzt werden können (DE-A 32 12 969, US 4 705 860, US 4 828 765, DE-A 33 47 378, WO 83/61 583). In dieser Literatur ist auch beschrieben, daß die Wirkung als Strömungsbeschleuniger verstärkt werden kann, wenn den quartären Ammoniumsalzen noch zusätzlich andere Elektrolyte zugegeben werden, wie etwa einfache anorganische Salze (NaCl, $CuCl_2$, $Na_2CO_3$) oder Salze organischer Säuren wie Na-Benzoat oder Na-Salicylat. Bevorzugt wird in solchen Fällen als zusätzlicher Elektrolyt ein Salz genommen, das das gleiche Anion enthält, wie die quartäre Ammoniumverbindung selbst. Mit solchen Mischungen erreicht man zwar eine Verstärkung der Wirkung als Strömungsbeschleuniger, doch entfalten diese Mischungen, genauso wie auch die quartären Ammoniumsalze selbst, ihre Wirkung meist nur in einem relativ engen Temperaturintervall.

Es wurde nun gefunden, daß man mit den im folgenden beschriebenen Mischungen von Salzen, die sowohl $\beta$-Hydroxynaphthoat als auch Salicylat enthalten, eine Wirkung als Strömungsbeschleuniger über einen sehr viel breiteren Temperaturbereich erreicht im Vergleich zu den bisher beschriebenen Mischungen.

Gegenstand der Erfindung sind somit Mischungen von organischen Ammoniumsalzen bestehend aus einem quaternären Ammoniumsalz der Formel 1

$R^1 - K^{\oplus}$ 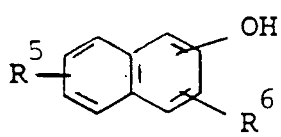 (1)

worin K eine Gruppe der Formel

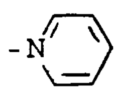 oder 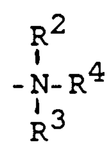 ,

| | |
|---|---|
| $R^1$ | $C_{12}$-$C_{26}$-Alkyl, $C_{12}$-$C_{26}$-Alkenyl, $C_{12}$-$C_{26}$-Fluoralkyl, $C_{12}$-$C_{26}$-Fluoralkenyl oder eine Gruppe der Formeln |

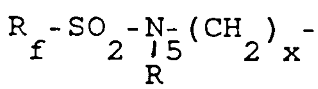 , 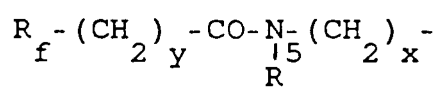

| | |
|---|---|
| | oder $R_f$-$(CH_2)_yB(CH_2)_x$- |
| $R^2$ und $R^3$ | $C_1$-$C_3$-Alkyl, vorzugsweise Methyl, |
| $R^4$ | $C_1$-$C_4$-Alkyl und für den Fall $R^1$ = Alkyl oder Alkenyl auch eine Gruppe der Formel $-(C_2H_4O)_zH$ |
| $R_f$ | $C_8$-$C_{20}$-Fluoralkyl oder $C_8$-$C_{20}$-Fluoralkenyl, |
| $R^5$ | Wasserstoff, Methyl oder Ethyl. |
| $R^6$ | $-COO^{\ominus}$ oder $-SO_3^{\ominus}$, |
| B | Sauerstoff oder Schwefel |
| x | eine ganze Zahl von 2 bis 6, |
| y | 0, 1 oder 2 |

und z Zahlen von 1 bis 4 bedeuten,
wobei für den Fall, daß K eine Gruppe der Formel

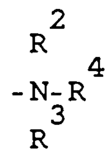

und $R^4$ eine Gruppe der Formel $-(C_2H_4O)_2H$ bedeutet, diese quartären Ammoniumsalze auch in einer Menge von 20 bis 80 Gew.-% durch die entsprechenden Aminsalze der Formel

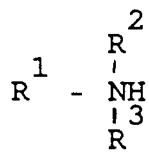

ersetzt sein können,
und zusätzlich ein Alkalisalz eines Anions der Formel 2

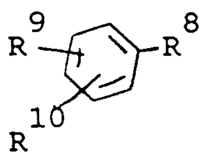

(2)

worin $R^8$ $-COO^{\ominus}$ oder $-SO_3^{\ominus}$, $R^9$ Wasserstoff, Hydroxyl, $NO_2$, Fluor, Chlor, Brom oder Jod und $R^{10}$ $C_1$-$C_5$-Alkyl, $C_2$-$C_5$-Alkenyl oder $C_1$-$C_5$-Alkoxy bedeuten, in einer Menge von 30 bis 70, vorzugsweise 40 bis 60, besonders bevorzugt 50 Mol-%, bezogen auf die vorhandene Gesamtmenge des Anions in der Verbindung der Formel 1.

Die quaternären Ammonium- und Aminsalze enthalten gemäß der obigen Formel äquivalente Mengen an Kationen und Anionen. Es ist jedoch auch möglich, daß eines der beiden Ionen im Überschuß vorliegt. Die fehlende Menge des entsprechenden Gegenions wird dann durch Gegenionen anderer Art, etwa Na- oder Cl-Ionen, ausgeglichen.

Bevorzugt sind solche Mischungen, in denen K eine Gruppe der Formel

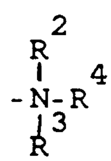

und A β-Hydroxynaphthoat bedeuten.

Insbesondere bevorzugt sind solche Mischungen, die quaternäre Ammoniumsalze der Formel

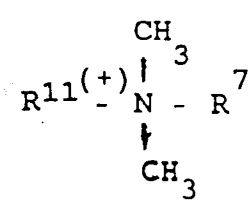

β-Hydroxynaphthoat$^{(-)}$

und Aminsalze der Formel

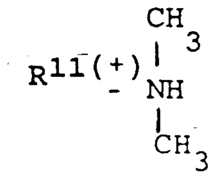 β-Hydroxynaphthoat(-)

enthalten, wobei $R^{11}$ $C_{14}$-$C_{22}$-Alkyl, $R^7$ Methyl oder die Gruppe $-(C_2H_4O)_zH$ und z Zahlen von 1 bis 4 bedeuten. Diese Mischungen enthalten vorzugsweise 20 bis 80 Gew.-% des quartären Ammonium-Salzes und entpsrechend 80 bis 20 Gew.-% des Aminsalzes. Bei den Anionen der Formel 2 ist das Salicylat-Anion bevorzugt.

Die Herstellung der Mischungen aus quaternären Ammoniumsalzen und Aminsalzen erfolgt nach bekannten Verfahren durch Alkylierung der zugrundeliegenden tertiären Amine beispielsweise mit Alkylhalogeniden oder Di-alkyl-sulfat. Die Herstellung der quaternären Ammoniumsalze, worin $R^4$ einen Polyoxyethylenrest darstellt, erfolgt durch oxalkylierende Quaternierung der tertiären Amine nach bekannten Verfahren (EP-A 379 260, US-A 2 836 517, US-A 1 223 730, US-A 3 223 718, US-A 2 897 170).

Bei den so erhaltenen Salzen, die als Anion ein Halogenid oder Methosulfat-Ion enthalten, werden diese Ionen nach bekannten Verfahren durch Ionenaustausch gegen das Ion der Formel

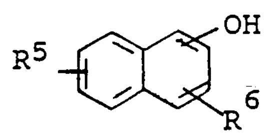

ausgetauscht, beispielsweise durch Umsetzung von β-Hydroxynaphthoesäure mit dem Hydroxid der quaternären Ammoniumverbindung. Dieses Hydroxid wiederum kann aus dem entsprechenden Ammoniumhalogenid mit Silberhydroxid gewonnen werden. Bevorzugt stellt man die Naphthoat-Salze her durch doppelte Umsetzung von Tetraalkylammonium-halogeniden mit Alkalihydroxid und Naphthoesäure in einem 2-Phasen-System aus Wasser und einem mit Wasser nicht mischbaren Lösemittel wie in DE-A 35 16 843 beschrieben.

Zu diesen Mischungen von quaternären Ammoniumsalzen und gegebenenfalls entsprechenden Aminsalzen bzw. deren wäßrigen Lösungen wird ein Alkalisalz eines Anions der Formel

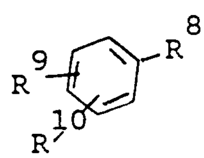

vorzugsweise Natriumsalicylat zugegeben in Mengen von 50 Mol-% bezogen auf die Gesamtmenge des Ions der Formel

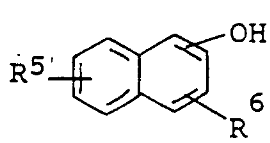

gerechnet als freie Säure. Das Alkalisalz des Anions der Formel (2) kann in fester Form oder als wäßrige Lösung zu der Lösung der Salze der Formel 1 zugegeben werden. Insgesamt sollen die wäßrigen

Lösungen bevorzugt 30 bis 40 Gew.-% an Feststoff enthalten. Für die Anwendung als Strömungsbeschleuniger in turbulent strömenden wäßrigen Medien, beispielsweise in Kühl- oder Heizkreisläufe, werden diese Lösungen weiter verdünnt auf eine Feststoffkonzentration von 0,01 bis 5, vorzugsweise 0,05 bis 1 Gew.-%.

Wie oben beschrieben, erhält man eine Wirkung als Strömungsbeschleuniger über einen sehr breiten Temperaturbereich mit Salzen, die β-Hydroxynaphthoat und Salicylat enthalten. Dieses Prinzip gilt allgemein auch für andere Kombinationen von quartären Ammonium-Salzen, wie sie in dem eingangs aufgeführten Stand der Technik beschrieben sind, mit anderen Salzen. Die jeweiligen Anionen sind dabei so auszuwählen, daß die Kombination des einen Anions mit dem quartären Ammonium-kation eine Wirksamkeit als Strömungsbeschleuniger in einem relativ niedrigen Temperaturbereich zeigt und die Kombination des anderen Anions mit dem gleichen quartären Ammonium-Kation diese Wirkung in einem relativ hohen Temperaturbereich zeigt.

Derartige Mischungen sind in einem größeren Temperaturbereich als Strömungsbeschleuniger wirksam verglichen mit den Einzelkomponenten und zwar über den gesamten Temperaturbereich der beiden Einzelkomponenten zusammengenommen. Weiterhin wird die Wirksamkeit der Reibungsminderung, d.h. maximal erzielbare Strömungsgeschwindigkeit, bevor die Reibungsminderung zusammenbricht, in der unteren Hälfte des Temperaturbereiches stark verbessert.

**Herstellung von Octadecyldimethyloxyethylammonium-β-hydroxynaphthoat (EO-OBON)**

In einem 1 l Laborglasautoklaven werden 181,6 g (0,60 mol; Alkalizahl: 185,4 mg KOH/g) Octadecyldimethylamin (tert. N 99,4 eq-%), 114,6 g (0,60 mol; Säurezahl: 293,3 mg KOH/g) β-Hydroxynaphthoesäure, 194,4 g (10,8 mol) demineralisiertes Wasser und 60,4 g (10 % m/m) Isopropanol vorgelegt. Nach zweimaligem Spülen mit Stickstoff wird die Reaktionsmischung auf 75 °C erwärmt. Nach Entspannen des dabei aufgebauten Druckes werden unter Rühren (ca. 500 U/min) 59,2 ml (1,20 mol) Ethylenoxid über eine Druckschleuse innerhalb von 2 Stunden kontinuierlich so zudosiert, daß die Innentemperatur nicht über 80 °C steigt. Während der Ethylenoxid-Zugabe steigt der Innendruck auf maximal 1,6 bar an.

Nach einer Reaktionszeit von weiteren 8 Stunden wird entspannt und das Produkt im Vakuum-Rotationsverdampfer andestilliert (Badtemperatur 70 °C). Man destilliert insgesamt 39 g ab und erhält so 591 g einer braungelben Lösung (bei längerem Stehenlassen bei Raumtemperatur Bildung eines Niederschlags), die 59 % Feststoffe, 5,5 % Isopropanol, 4,3 % Ethylenglykol, 0,2 % Diethylenglykol und 30 % Wasser enthält. Der Gehalt an Ethylenoxid beträgt 1,3 ppm. Der durch Epton-Titration bestimmte Quaternierungsgrad (Titration bei pH 1 - 2 und pH ≧ 10) beträgt 47,4 %.

Zur Herstellung der Mischung von EO-OBON mit Natriumsalicylat versetzt man das nach dem Andestillieren erhaltene Produkt (591 g) bei einer Temperatur von 50 °C mit 48 g (0,30 mol, 50 mol-% bezogen auf eingesetzte β-Hydroxynaphthoesäure) Natriumsalicylat und rührt 1 Stunde bei dieser Temperatur.

**Meßapparatur**

Zur Messung der Reibungsminderung wird eine geschlossene Strömungsapparatur benutzt, in der die Testlösung im Kreislauf umgepumpt wird. Die Meßstrecke zur Bestimmung des Druckverlustes $\Delta P$ beträgt 1 m, der Rohrdurchmesser 6 mm und das Verhältnis der Einlauflänge 1 zum Rohrdurchmesser d (1/d) 280. Die Bestimmung der Strömungsgeschwindigkeit V erfolgt mit einem induktiven Durchflußmesser. Die gesamte Testapparatur (gesamtes Lösungsvolumen) läßt sich von 10 °C bis 150 °C auf ±3 °C temperieren.

Vor Durchführung der Messungen wurden die Lösungen jeweils mindestens für 2 Stunden bei den Meßtemperaturen in der Apparatur umgepumpt. Vor Durchführung der Messungen bei Temperaturen kleiner gleich 45 °C wurden die Lösungen über Nacht oder nach Möglichkeit übers Wochenende durch Umpumpen bei den jeweiligen Temperaturen temperiert.

Nach Einfüllen der Lösungen in die Meßapparatur wurden die Lösungen generell unter Umpumpen auf mindestens 120 °C erhitzt und erst dann auf die jeweilige Meßtemperatur wieder abgekühlt.

Die Untersuchungen der genannten Mischungen auf ihre Wirksamkeit als Strömungsbeschleuniger erfolgte in der üblichen Art, indem für die jeweilige wäßrige Lösung der Tenside der Druckabfall $\Delta P$ über die Strecke 1 bei Durchströmen eines Rohres mit dem Querschnitt d für verschiedene Strömungsgeschwindigkeiten V gemessen wurde.

Aus diesen Werten lassen sich die dimensionslosen Größen, Reibungswert $\lambda$ und die Reynoldszahl Re, berechnen:

$$\lambda = \frac{2d}{\rho V^2} \cdot \frac{\Delta P}{L}$$

$$Re = \frac{V \cdot d}{\nu}$$

wobei $\rho$ die Dichte und $\nu$ die kinematische Viskosität bedeuten. Üblicherweise werden für $\rho$ und $\nu$ jeweils die entsprechenden Werte des reinen Lösungsmittels, Wasser, eingesetzt. Die so erhaltenen Werte λ, Re, für die untersuchten Tensid-Lösungen wurden in der üblichen doppelt logaritmischen Auftragung λ gegen Re mit den entsprechenden Werten für reines Wasser, wiedergegeben durch

$1/\sqrt{\lambda} = 2 \log Re\sqrt{\lambda}-0{,}8$

verglichen. Eine Wirkung als SB oder auch Reibungsminderung liegt dann vor, wenn gilt:

$$\lambda_{H_2O} - \lambda_{SB} > 0,$$

bzw. die Größe der Reibungsminderung in Prozent berechnet sich nach:

$$\alpha = \%\ \text{Reibungsminderung} = \frac{\lambda_{H_2O} - \lambda_{SB}}{\lambda_{H_2O}} \times 100$$

Der Grad der Wirksamkeit einer Tensidlösung als SB soll im folgenden durch die Größe von $Re_{max}$ gekennzeichnet werden; demnach besitzt eine Tensidlösung mit $Re_{max}$ = 20 000 eine bessere Wirksamkeit als SB als eine Tensidlösung mit $Re_{max}$ = 10 000. Der zugehörige $\alpha$-Wert soll mit $\alpha_{max}$ gekennzeichnet werden.

Die Tabellen in den folgenden Beispielen zeigen die gemessenen Werte für die Durchflußgeschwindigkeit V, für $Re_{max}$ und $\alpha_{max}$ bei verschiedenen Temperaturen.

**Beispiel 1**

Tabelle 1

| Octadecyldimethylhydroxyethylammonium-3-hydroxy-2-naphthoat, Konzentration: 1000 ppm | | | |
|---|---|---|---|
| T/°C | $V_{max}$ (m/s) | $Re_{max}$ ($\cdot 10^{-3}$) | $\alpha_{max}$ (% Reibungsminderung) |
| 45 | 0.92 | 9300 ± 950 | 60 ± 3 |
| 60 | 1.36 | 17000 ± 1700 | 75 ± 4 |
| 75 | 1.93 | 29500 ± 3000 | 77 ± 4 |
| 90 | 3.51 | 64800 ± 6500 | 82 ± 4 |
| 105 | 4.39 | 94100 ± 9500 | 84 ± 4 |
| 115 | 4.77 | 110400 ± 11000 | 81 ± 4 |
| 123 | 3.69 | 90900 ± 9100 | 80 ± 4 |

Tabelle 2

| Octadecyldimethylhydroxyethylammonium-3-hydroxy-2-naphthoat, Konzentration 1000 ppm + 2 mmol Natriumsalicylat | | | |
|---|---|---|---|
| T/°C | $V_{max}$ (m/s) | $Re_{max}$ ($10^{-3}$) | $\alpha_{max}$ (% Reibungsminderung) |
| 30 | 3.13 | 23600 ± 2500 | 75 ± 4 |
| 46 | 3.68 | 36900 ± 3700 | 78 ± 4 |
| 60 | 2.77 | 34600 ± 3500 | 77 ± 4 |
| 75 | 2.78 | 42400 ± 4500 | 74 ± 4 |
| 90 | 3.45 | 63800 ± 6500 | 79 ± 4 |
| 105 | 4.40 | 94100 ± 9500 | 79 ± 4 |
| 115 | 4.69 | 108800 ± 11000 | 84 ± 4 |
| 125 | 3.09 | 76800 ± 8000 | 56 ± 3 |

Tabelle 1 zeigt die Werte für ein quaternäres Ammonium-$\beta$-hydroxynaphthoat ohne Zusatz von Salicylat, während in Tabelle 2 die entsprechenden Werte für die gleiche Verbindung, aber mit einem Zusatz von Na-salicylat angegeben sind. Diese Werte sind in Figur 1 und 2 graphisch dargestellt.

Der Vergleich der unteren Kurven der beiden Figuren, die die Durchflußgeschwindigkeit darstellen, zeigt, daß durch die Zugabe von Salicylat die Höhe der Reibungsminderung (Durchflußgeschwindigkeit) bei hohen Temperaturen erhalten bleibt und darüberhinaus auch auf den Bereich niedriger Temperaturen ausgedehnt wird. Dieser Befund war überraschend, da ja in beiden Fällen die Konzentration des quaternären Ammonium-Kations gleich bleibt, der Effekt der Erhöhung der Reibungsminderung (Durchflußgeschwindigkeit) bei niederen Temperaturen also auf die Mischung verschiedener Kationen zurückzuführen ist. Na-Salicylat allein hat aber andererseits für sich allein genommen keine Wirkung als Strömungsbeschleuniger.

Tabelle 3

| Octadecyl-dimethylhydroxyethyl-ammonium-3-hydroxy-2-naphthoat Konzentration 1000 ppm + 0,5 mmol Natriumsalicylat | | | |
|---|---|---|---|
| T/°C | $V_{max}$ (m/s) | $Re_{max}$ ($10^{-3}$) | $\alpha_{max}$ (% Reibungsminderung) |
| 20 | 1.32 | 7940 | 57.6 |
| 30 | 1.59 | 11900 | 63.0 |
| 50 | 2.36 | 25300 | 72.0 |
| 70 | 2.36 | 34000 | 74.5 |
| 90 | 2.99 | 54700 | 78.26 |
| 110 | 4.7 | 105000 | 78.82 |
| 120 | 4.77 | 115000 | 68.91 |
| 125 | 3.89 | 97000 | 56.94 |

Tabelle 4

| Octadecyl-dimethylhydroxyethyl-ammonium-3-hydroxy-2-naphthoat Konzentration 1000 ppm + 2 mmol Natriumsalicylat | | | |
|---|---|---|---|
| T/° C | $V_{max}$ (m/s) | $Re_{max}$ ($10^{-3}$) | $\alpha_{max}$ (% Reibungsminderung) |
| 21 | 6.83 | 42000 | 78.53 |
| 30 | 6.44 | 48500 | 78.28 |
| 50 | 5.55 | 59800 | 78.87 |
| 70 | 4.62 | 42300 | 78.49 |
| 90 | 3.0 | 55000 | 76.68 |
| 110 | 4.57 | 103000 | 74.98 |
| 120 | 3.28 | 79300 | 59.90 |

Die Tabellen 3 und 4 zeigen, wie sich unterschiedliche Konzentrationen an Na-Salicylat auf den beschriebenen Effekt auswirken. Aus den Tabellen geht hervor, daß eine zu hohe Konzentration an Na-Salicylat zwar die Höhe der Reibungsminderung bei niedrigen Temperaturen stark verbessert, bei hohen Temperaturen aber verschlechtert. Für die Anwendung in der Praxis ist jedoch eine möglichst gleichbleibende Reibungsminderung über den gesamten Temperaturbereich wünschenswert.

In den folgenden Beispielen 2 bis 4 wird für andere quaternäre Ammoniumsalze gezeigt, daß durch den Zusatz von Na-Salicylat der Bereich, in dem eine Reibungsminderung auftritt, zu tiefen Temperaturen verbreitert wird.

**Beispiel 2**

**Tabelle 5**

Octadecyl-trimethylammonium-3-hydroxy-2-naphthoat

Konzentration: 500 ppm

| T/°C | $V_{max}$ (m/s) | $Re_{max}$ ($10^{-3}$) | $\alpha_{max}$ (% Reibungsminderung) |
|---|---|---|---|
| 61 | 0.86 | 10900 | 71.02 |
| 70 | 1.24 | 17800 | 74.43 |
| 80 | 2.29 | 50200 | 77.53 |
| 100 | 2.73 | 55100 | 79.23 |
| 110 | 3.39 | 75300 | 80.31 |
| 120 | 3.37 | 81000 | 81.54 |
| 127 | 1.35 | 34100 | 68.98 |

Tabelle 6

| Octadecyl-trimethylammonium-3-hydroxy-2-naphthoat Konzentration: 500 ppm + $10^{-3}$ mol/l Natrium-Salicylat | | | |
|---|---|---|---|
| T/°C | $V_{max}$ (m/s) | $Re_{max}$ ($10^{-3}$) | $\alpha_{max}$ (% Reibungsminderung) |
| 44 | 0.45 | 4340 | 47.01 |
| 50 | 1.03 | 11000 | 66.58 |
| 60 | 2.47 | 30900 | 73.77 |
| 70 | 2.36 | 33900 | 75.80 |
| 80 | 3.18 | 51900 | 76.31 |
| 100 | 3.52 | 71700 | 81.86 |
| 110 | 3.40 | 70000 | 82.93 |
| 120 | 2.64 | 63700 | 74.07 |
| 125 | 1.56 | 38900 | 18.58 |

**Beispiel 3**

**Tabelle 7**

Hexadecyl-trimethylammonium-3-hydroxy-2-naphthoat
Konzentration: 500 ppm

| T/°C | $V_{max}$ (m/s) | $Re_{max}$ ($10^{-3}$) | $\alpha_{max}$ (% Reibungsminderung) |
|---|---|---|---|
| 40 | 1.02 | 9300 | 58.88 |
| 60 | 2.28 | 28300 | 72.63 |
| 80 | 3.35 | 54300 | 77.79 |
| 90 | 3.29 | 60300 | 77.00 |
| 100 | 2.58 | 52800 | 77,72 |
| 104 | 1.85 | 39400 | 76.99 |
| 107 | 1.78 | 39000 | 54.71 |

Tabelle 8

| Hexadecyl-trimethylammonium-3-hydroxy-2-naphthoat Konzentration: 500 ppm + $10^{-3}$ mol/l Natrium-Salicylat | | | |
|---|---|---|---|
| T/° C | $V_{max}$ (m/s) | $Re_{max}$ ($10^{-3}$) | $\alpha_{max}$ (% Reibungsminderung) |
| 25 | 0.78 | 5270 | 58.11 |
| 40 | 1.56 | 14300 | 68.78 |
| 60 | 2.80 | 34900 | 75.81 |
| 80 | 3.25 | 52900 | 77.78 |
| 90 | 3.26 | 59900 | 78.22 |
| 100 | 2.49 | 51000 | 77.63 |
| 105 | 1.83 | 39100 | 44.32 |

**Beispiel 4**

Tabelle 9

Docosyl-trimethylammonium-3-hydroxy-2-naphthoat
Konzentration: 1000 ppm

| T/°C | $V_{max}$ (m/s) | $Re_{max}$ ($10^{-3}$) | $\alpha_{max}$ (% Reibungsminderung) |
|---|---|---|---|
| 80 | 1.0 | 16500 | 60 |
| 90 | 4.5 | 83000 | 75 |
| 100 | 4.0 | 81700 | 77 |
| 110 | 3.3 | 74000 | 78 |
| 120 | 3.0 | 73200 | 78 |
| 130 | 2.7 | 71200 | 80 |
| 140 | 2.5 | 70700 | 82 |
| 145 | 2.5 | 73100 | 81 |
| 150 | 1.5 | 45300 | 65 |

Tabelle 10

| Docosyl-trimethylammonium-3-hydroxy-2-naphthoat Konzentration: 1000 ppm + 1,8 mmol Natrium-Salicylat | | | |
|---|---|---|---|
| T/°C | $V_{max}$ (m/s) | $Re_{max}$ ($10^{-3}$) | $\alpha_{max}$ (% Reibungsminderung) |
| 50 | 2.21 | 23600 | 73.01 |
| 60 | 2.38 | 29700 | 76.57 |
| 70 | 3.06 | 43700 | 76.99 |
| 80 | 4.14 | 67600 | 77.17 |
| 95 | 3.59 | 69600 | 77.57 |
| 110 | 3.0 | 67100 | 78.2 |
| 125 | 2.69 | 67300 | 77.63 |
| 130 | 3.0 | 77500 | 73.65 |
| 135 | 2.69 | 71600 | 82.7 |
| 140 | 2.67 | 73500 | 81.8 |
| 145 | 2.0 | 56900 | 82.65 |

## Patentansprüche

**1.** Mischungen von organischen Ammoniumsalzen bestehend aus einem quartären Ammoniumsalz der Formel (1)

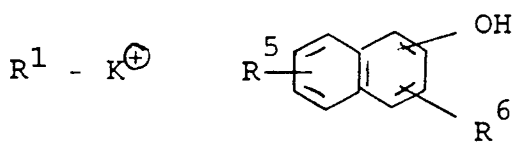

(1)

worin K eine Gruppe der Formel

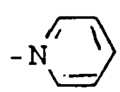 oder 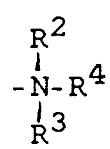 ,

| | |
|---|---|
| $R^1$ | $C_{12}$-$C_{26}$-Alkyl, $C_{12}$-$C_{26}$-Alkenyl, $C_{12}$-$C_{26}$-Fluoralkyl, $C_{12}$-$C_{26}$-Fluoralkenyl oder eine Gruppe der Formeln |

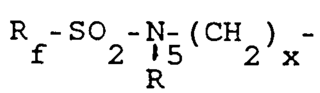 , 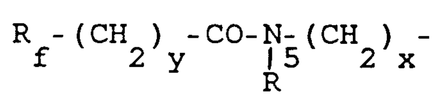

| | |
|---|---|
| | oder $R_f$-$(CH_2)_yB(CH_2)_x$- |
| $R^2$ und $R^3$ | $C_1$-$C_3$-Alkyl, vorzugsweise Methyl, |
| $R^4$ | $C_1$-$C_4$-Alkyl und für den Fall $R^1$ = Alkyl oder Alkenyl auch eine Gruppe der Formel $-(C_2H_4O)_zH$ |
| $R_f$ | $C_8$-$C_{20}$-Fluoralkyl oder $C_8$-$C_{20}$-Fluoralkenyl, |
| $R^5$ | Wasserstoff, Methyl oder Ethyl. |
| $R^6$ | $-COO^{\ominus}$ oder $-SO_3^{\ominus}$ |

| | |
|---|---|
| B | Sauerstoff oder Schwefel |
| x | eine ganze Zahl von 2 bis 6, |
| y | 0, 1 oder 2 |

und z Zahlen von 1 bis 4 bedeuten,
wobei für den Fall, daß K eine Gruppe der Formel

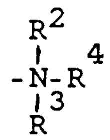

und $R^4$ eine Gruppe der Formel $-(C_2H_4O)_2H$ bedeutet, diese quartären Ammoniumsalze auch in einer Menge von 20 bis 80 Gew.-% durch die entsprechenden Aminsalze der Formel

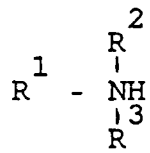

ersetzt sein können,
und zusätzlich ein Alkalisalz eines Anions der Formel 2

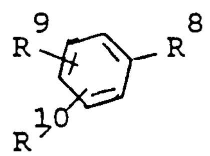

(2)

worin $R^8$ $-COO^{\ominus}$ oder $-SO_3^{\ominus}$, $R^9$ Wasserstoff, Hydroxyl, $NO_2$, Fluor, Chlor, Brom oder Jod und $R^{10}$ $C_1$-$C_5$-Alkyl, $C_2$-$C_5$-Alkenyl oder $C_1$-$C_5$ Alkoxy bedeuten, in einer Menge von 30 bis 70, vorzugsweise 40 bis 60, besonders bevorzugt 50 Mol%, bezogen auf die vorhandene Gesamtmenge des Anions in der Verbindung der Formel 1.

**2.** Mischungen nach Anspruch 1 enthaltend eine Verbindung der Formel 1, worin K eine Gruppe der Formel

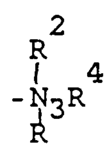

und
A $\beta$-Hydroxynaphthoat bedeutet.

**3.** Mischungen nach Anspruch 1 bestehend aus quaternären Ammoniumsalzen und Aminsalzen der Formeln

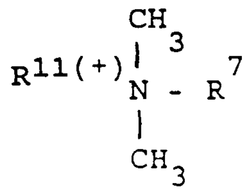

β-Hydroxynaphthoat⊖

und

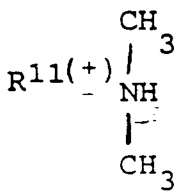

β-Hydroxynaphthoat⊖

wobei $R^{11}$ $C_{14}$-$C_{22}$-Alkyl, $R^7$ Methyl oder die Gruppe $-(C_2H_4O)_x$ H und x Zahlen von 1 bis 4 bedeuten.

4. Mischungen nach Anspruch 1, dadurch gekennzeichnet, daß das Anion der Formel 2 Salicylat ist.

5. Wäßrige Lösungen enthaltend 0,01 bis 5 Gew.-% einer Mischung nach Anspruch 1.

6. Verwendung der Mischungen nach Anspruch 1 als Strömungsbeschleuniger.

**Claims**

1. A mixture of organic ammonium salts composed of a quaternary ammonium salt of the formula (1)

$R^1 - K^{\oplus}$ 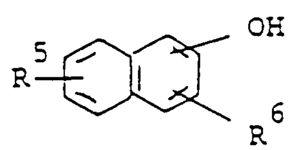 (1)

where K is a group of the formula

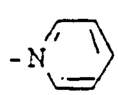 or 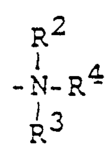 ,

| | |
|---|---|
| $R^1$ | is $C_{12}$-$C_{26}$-alkyl, $C_{12}$-$C_{26}$-alkenyl, $C_{12}$-$C_{26}$-fluoroalkyl, $C_{12}$-$C_{26}$-fluoroalkenyl or a group of the formulae |

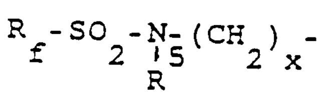 , 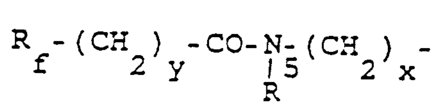

| | |
|---|---|
| | or $R_f$-$(CH_2)_yB(CH_2)_x$-, |
| $R^2$ and $R^3$ | is $C_1$-$C_3$-alkyl, preferably methyl, |
| $R^4$ | is $C_1$-$C_4$-alkyl and, for the case where $R^1$ = alkyl or alkenyl, also a group of the formula $-(C_2H_4O)_zH$, |
| $R_f$ | is $C_8$-$C_{20}$-fluoroalkyl or $C_8$-$C_{20}$-fluoroalkenyl, |

| | |
|---|---|
| $R^5$ | is hydrogen, methyl or ethyl, |
| $R^6$ | is $-COO^{\ominus}$ or $-SO_3^{\ominus}$, |
| B | is oxygen or sulfur, |
| x | is an integer from 2 to 6, |
| y | is 0, 1 or 2, |

and z is numbers from 1 to 4,
it being possible, in the case where K is a group of the formula

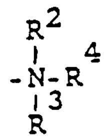

and $R^4$ is a group of the formula $-(C_2H_4O)_2H$, for said quaternary ammonium salts also to be replaced in a quantity of 20 to 80% by weight by the corresponding amine salts of the formula

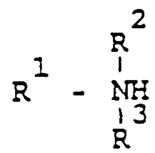

and additionally an alkali metal salt of an anion of the formula 2

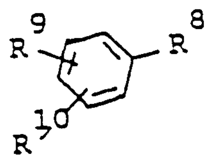

(2)

where $R^8$ is $-COO^{\ominus}$ or $-SO_3^{\ominus}$, $R^9$ is hydrogen, hydroxyl, $NO_2$, fluorine, chlorine, bromine or iodine and $R^{10}$ is $C_1$-$C_5$-alkyl, $C_2$-$C_5$-alkenyl or $C_1$-$C_5$-alkoxy, in a quantity of 30 to 70, preferably 40 to 60, particularly preferably 50, mol %, based on the total amount of the anion present in the compound of the formula 1.

2. A mixture as claimed in claim 1 containing a compound of the formula 1, where K is a group of the formula

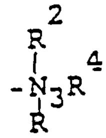

and A is $\beta$-hydroxynaphthoate.

3. A mixture as claimed in claim 1 composed of quaternary ammonium salts and amine salts of the formula

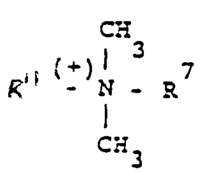

$\beta$-hydroxynaphthoate$^{\ominus}$

and

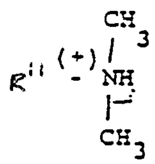

$\beta$-hydroxynaphthoate$^{\ominus}$,

where $R^{11}$ is $C_{14}$-$C_{22}$-alkyl, $R^7$ is methyl or the group $-(C_2H_4O)_xH$ and x is numbers from 1 to 4.

**4.** A mixture as claimed in claim 1, wherein the anion of the formula 2 is salicylate.

**5.** An aqueous solution containing 0.01 to 5% by weight of the mixture as claimed in claim 1.

**6.** The use of the mixture as claimed in claim 1 as flow accelerator.

**Revendications**

**1.** Mélanges de sels organiques d'ammonium consistant en un sel d'ammonium quaternaire de formule

$R^1$ - $K^{\oplus}$ 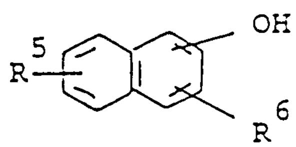 (1)

dans laquelle K représente un groupe de formule

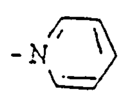

ou

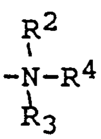

$R^1$ représente un groupe alkyle en $C_{12}$ à $C_{26}$, alcényle en $C_{12}$ à $C_{26}$, fluoralkyle en $C_{12}$ à $C_{26}$, fluoralcényle en $C_{12}$ à $C_{26}$, ou un groupe répondant aux formules

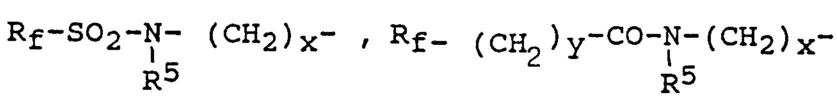

ou $R_f$- $(CH_2)_yB(CH_2)_x$-

$R^2$ et $R^3$ représentent chacun un groupe alkyle en $C_1$ à $C_3$, avantageusement un groupe méthyle,

$R^4$ représente un groupe alkyle en $C_1$ à $C_4$ et, si $R^1$ représente un groupe alkyle ou alcényle, $R^4$ peut également représenter un groupe de formule $-(C_2H_4O)_zH$,

$R_f$ représente un groupe fluoralkyle en $C_8$ à $C_{20}$ ou fluoralcényle en $C_8$ à $C_{20}$,

$R^5$ représente un atome d'hydrogène, un groupe méthyle ou éthyle,

$R^6$ représente $-COO^{\ominus}$ ou $-SO_3^{\ominus}$
B représente un atome d'oxygène ou de soufre,
x est un nombre entier valant 2 à 6,
y est un nombre valant 0, 1, ou 2,
et z est un nombre valant 1 à 4,
et, si K représente un groupe de formule

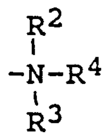

et si R' représente un groupe de formule $-(C_2H_4O)_2H$,

ces sels d'ammonium quaternaire peuvent également être remplacés, en une quantité de 20 à 80 % en poids, par les sels d'amines correspondantes de formule

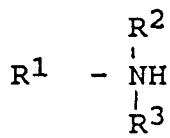

et, en outre, en un sel alcalin d'un anion de formule 2

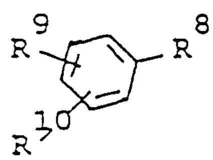

(2)

dans laquelle $R^8$ représente - $COO^{\ominus}$ ou $-SO_3^{\ominus}$; $R^9$ représente un atome d'hydrogène, un groupe hydroxyle, $NO_2$, un atome de fluor, de chlore, de brome ou d'iode, et $R^{10}$ représente un groupe alkyle en $C_1$ à $C_5$, alcényle en $C_2$ à $C_5$ ou alcoxy en $C_1$ à $C_5$, sels présents en une quantité de 30 à 70, avantageusement 40 à 60, de façon particulièrement préférée 50 moles %, par rapport à la quantité totale présente de l'anion du composé de formule 1.

**2.** Mélanges selon la revendication 1, contenant un composé de formule 1, dans laquelle K représente un groupe de formule

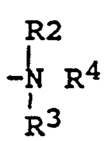

,

et A représente un groupe $\beta$-hydroxynaphtoate.

**3.** Mélanges selon la revendication 1, consistant en des sels d'ammonium quaternaire et en des sels d'amines répondant aux formules

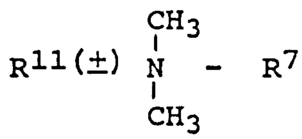 ß-hydroxynaphtoate

et,

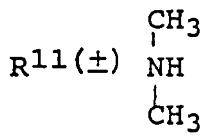 ß-hydroxynaphtoate

formules dans lesquelles $R^{11}$ représente un groupe alkyle en $C_{14}$ à $C_{22}$, $R^7$ représente un groupe méthyle ou le groupe $-C_2H_4O)_xH$ , et x est un nombre valant 1 à 4.

**4.** Mélanges selon la revendication 1, caractérisés en ce que l'anion de formule 2 est un anion salicylate.

**5.** Solutions aqueuses contenant 0,01 à 5 % en poids d'un mélange selon la revendication 1.

**6.** Utilisation des mélanges selon la revendication 1 à titre d'accélérateurs d'écoulements.

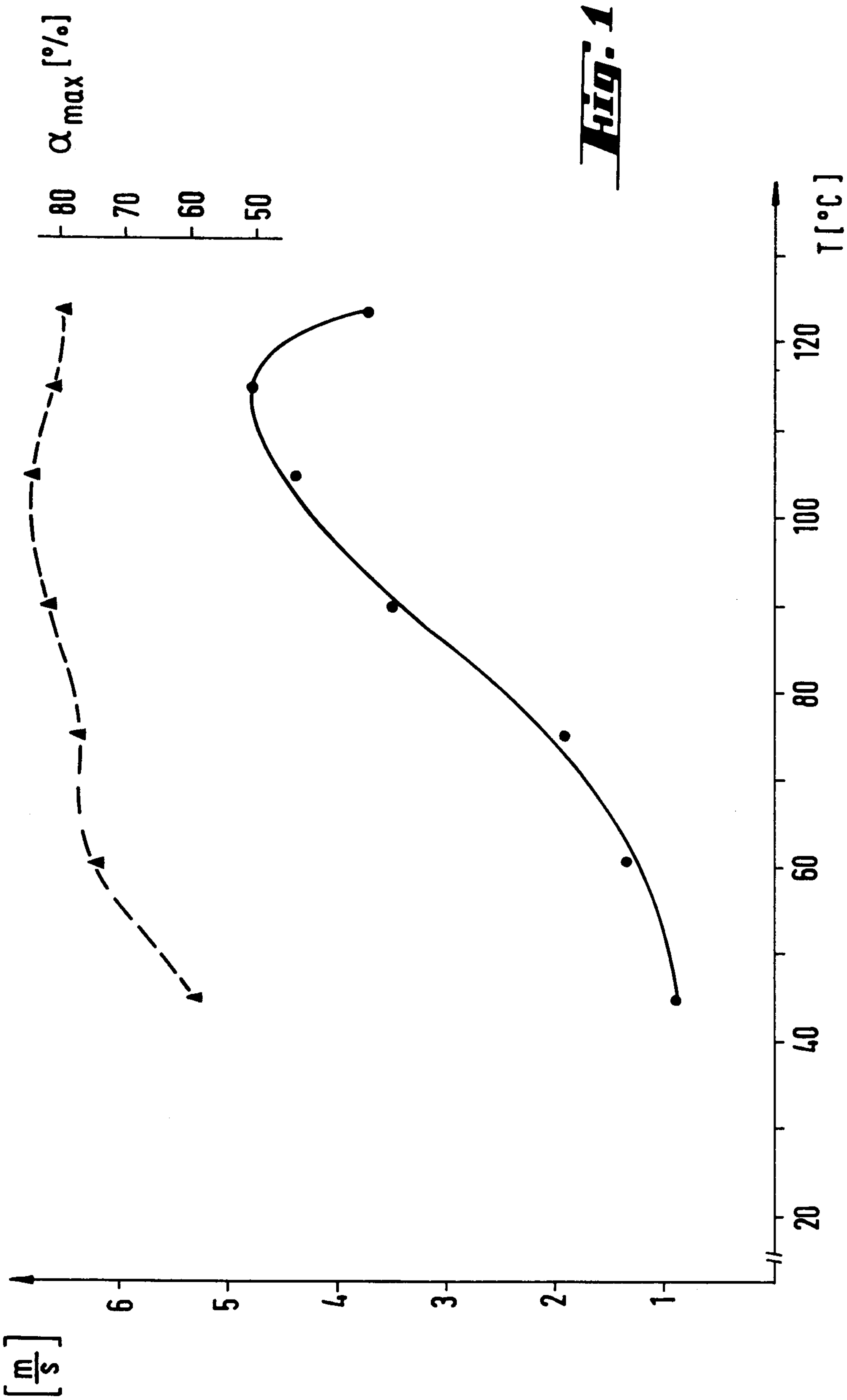


Fig. 1

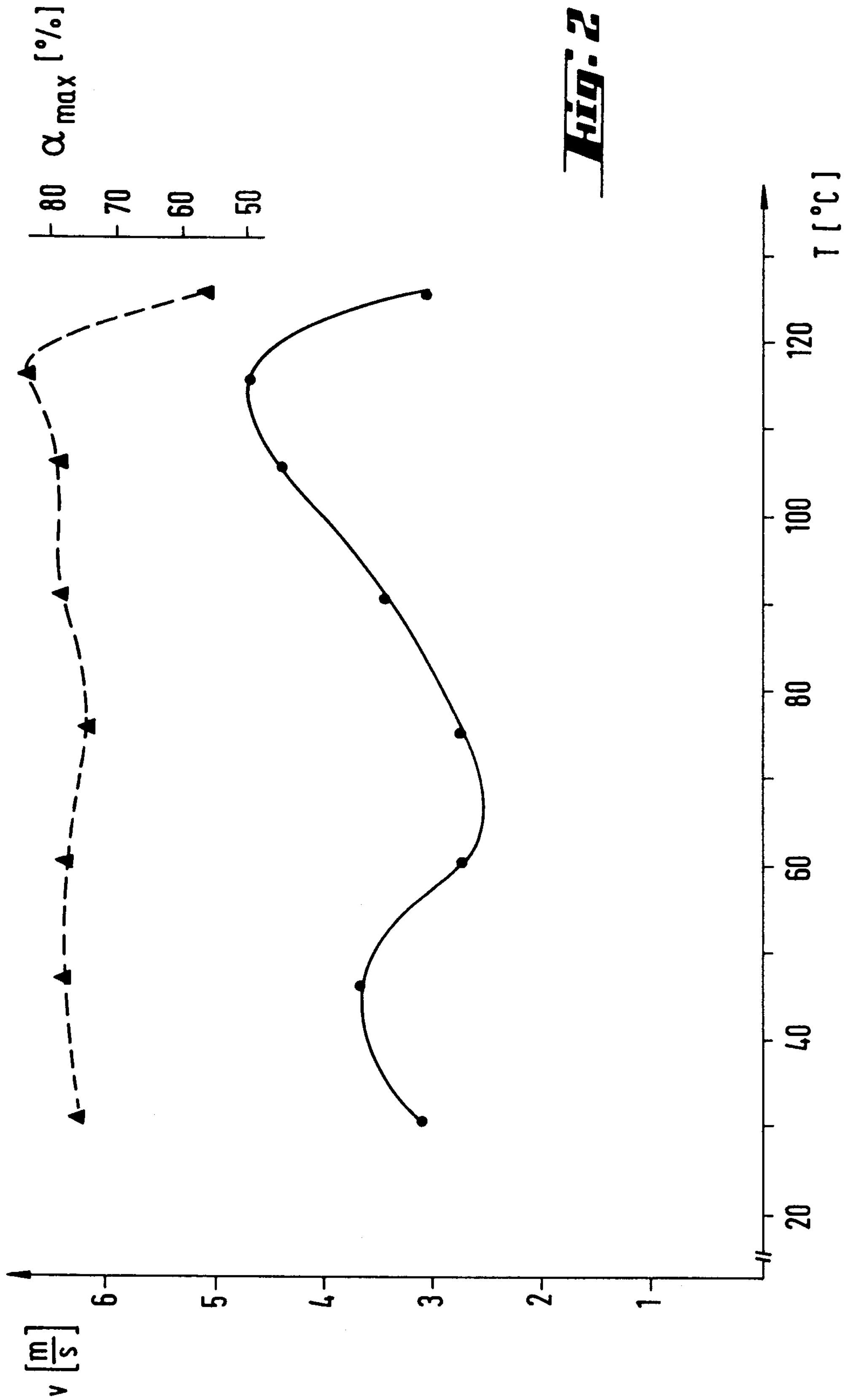


Fig. 2